# EUROPÄISCHE PATENTANMELDUNG

(11) **EP 4 074 682 A1**
(43) Veröffentlichungstag der Anmeldung: **19.10.2022**
(21) Anmeldenummer: 21168761.1
(22) Anmeldetag: 16.04.2021
(51) Int. Cl.: C07C 5/03, C07C 15/073, C07C 29/14, C07C 33/20, C07C 45/50, C07C 47/11

(54) **VERFAHREN ZUR HYDROXYMETHYLIERUNG UND HYDROFORMYLIERUNG VON ALKENEN MITTELS KUPFER-LIGAND-KOMPLEX**

(71) Anmelder: Evonik Operations GmbH, 45128 Essen (DE)
(72) Erfinder: WU, Xiao-Feng, 18059 Rostock (DE); MEYER, Tim, 18057 Rostock (DE); GENG, Hui-Qing, 18059 Rostock (DE); BÖRNER, Armin, 18059 Rostock (DE); FRANKE, Robert, 45772 Marl (DE)
(74) Vertreter: Evonik Patent Association

(57) **Zusammenfassung**

Verfahren zur Hydroxymethylierung und Hydroformylierung von Alkenen mittels Kupfer-Ligand-Komplex.

## Beschreibung

Die Erfindung betrifft ein Verfahren zur Hydroxymethylierung und Hydroformylierung von Alkenen mittels Kupfer-Ligand-Komplex.

In "Alternative Metals for Homogeneous Catalyzed Hydroformylation Reactions" R. Franke, M. Beller et al., Angew. Chem. Int. Ed. 2013, 52, 2852 - 2872 werden eine Reihe von Metallen beschrieben, welche zur Katalyse von Hydroformylierungsreaktionen eingesetzt werden. Bei den Metallen handelt es sich um: Ru, Ir, Pd, Pt, und Fe.

Die technische Aufgabe, welche der vorliegenden Erfindung zugrunde lag, ist die Bereitstellung eines Verfahrens, mit welchen Alkene hydroxymethyliert und/oder hydroformyliert werden können. In dem Verfahren soll ein anderes Metall zum Einsatz kommen als die Metalle Ru, Ir, Pd, Pt, und Fe. Mit diesem Verfahren soll eine gesteigerte Ausbeute erzielt werden.

Diese Aufgabe wird gelöst durch ein Verfahren nach Anspruch 1.

Verfahren umfassend die Verfahrensschritte:
a) Vorlegen eines Alkens;
b) Zugabe eines Liganden gemäß Formel **(I):** 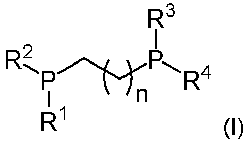 wobei
   R¹, R², R³, R⁴ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -(C₅-C₂₀)-Heteroaryl,
   und n für eine Zahl von 1 bis 8 steht;
c) Zugabe einer Verbindung, welche Cu umfasst;
d) Zuführen von CO und H₂;
e) Erwärmen des Reaktionsgemisches aus a) bis d), wobei das Alken zu einem Aldehyd und/oder Alkohol umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Der Begriff -(C₁-C₁₂)-Alkyl bzw. -O-(C₁-C₁₂)-Alkyl umfasst geradkettige und verzweigte Alkylgruppen mit 1 bis 12 Kohlenstoffatomen. Vorzugsweise handelt es sich dabei um -(C₁-C₈)-Alkylgruppen bzw. -O-(C₁-C₈)-Alkylgruppen, besonders bevorzugt um -(C₁-C₄)-Alkylgruppen bzw. -O-(C₁-C₄)-Alkylgruppen.

Der Begriff -(C₆-C₂₀)-Aryl und -O-(C₆-C₂₀)-Aryl umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Der Begriff -(C₅-C₂₀)-Heteroaryl umfasst im Sinne der vorliegenden Erfindung mono- oder polycyclische aromatische Kohlenwasserstoffreste, in denen ein oder mehrere C-Atome durch Heteroatome, vorzugsweise N, O oder S ersetzt sind. Hierbei ist N besonders bevorzugt. Beispiele hierfür sind unter anderem Pyridyl, 2-Pyridyl, Pyrimidyl, Imidazolyl, Thiopenyl, Furanyl, Pyrrolyl, Pyrazoly, Thiazolyl oder Isoxazolyl. Die mono- oder polycyclische aromatische Kohlenwasserstoffreste weisen 5 bis 20 Ringatome, bevorzugt 5 bis 14 Ringatome, insbesondere 5 und 6 Ringatome, auf.

In einer Variante des Verfahrens stehen R¹, R² für -(C₆-C₂₀)-Aryl.

In einer Variante des Verfahrens stehen R¹, R² für -Ph.

In einer Variante des Verfahrens stehen R¹, R³ für -(C₆-C₂₀)-Aryl.

In einer Variante des Verfahrens stehen R¹, R³ für -Ph.

In einer Variante des Verfahrens weist der Ligand eine der Strukturen (1), (2), (3) auf:

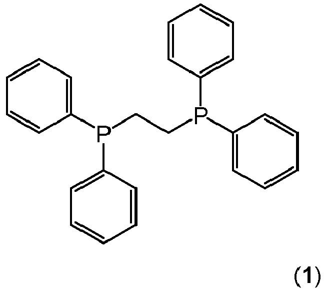

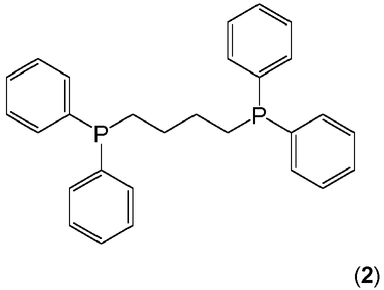

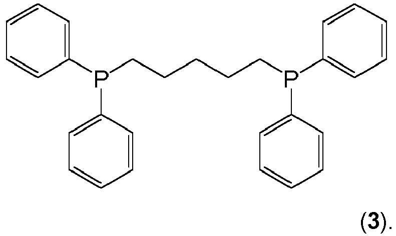

In einer Variante des Verfahrens ist die Verbindung, welche Cu umfasst, ausgewählt aus: CuCl₂, CuBr₂, Cu(OAc)₂, Cu(OTf)₂, CuCl, CuBr, CuI, Cul(MeSMe).

In einer Variante des Verfahrens erfolgt das Zuführen von CO in Verfahrensschritt d) mit einem Druck in dem Bereich von 0,5 bis 5 MPa (5 bis 50 bar).

In einer Variante des Verfahrens erfolgt das Zuführen von CO in Verfahrensschritt d) mit einem Druck in dem Bereich von 0,5 bis 3 MPa (5 bis 30 bar).

In einer Variante des Verfahrens erfolgt das Zuführen von H₂ in Verfahrensschritt d) mit einem Druck in dem Bereich von 1 bis 5 MPa (10 bis 50 bar).

In einer Variante des Verfahrens erfolgt das Zuführen von H₂ in Verfahrensschritt d) mit einem Druck in dem Bereich von 1 bis 3 MPa (10 bis 30 bar).

In einer Variante des Verfahrens erfolgt das Erwärmen des Reaktionsgemisches in Verfahrensschritt e) auf eine Temperatur in dem Bereich von 70 °C bis 150 °C.

In einer Variante des Verfahrens erfolgt das Erwärmen des Reaktionsgemisches in Verfahrensschritt e) auf eine Temperatur in dem Bereich von 80 °C bis 120 °C.

In einer Variante des Verfahrens umfasst das Verfahren den zusätzlichen Verfahrensschritt d'):
d') Zugabe einer Base.

In einer Variante des Verfahrens ist die Base ausgewählt aus: KO^{t}Bu, NaO^{t}Bu, KOMe, NaOEt, DBU, NEt₃.

In einer Variante des Verfahrens den zusätzlichen Verfahrensschritt d"):
d") Zugabe eines Lösungsmittels.

In einer Variante des Verfahrens ist das Lösungsmittel ausgewählt ist aus: Cyclohexan, Toluol, CH₃CN, DMSO, DMF, ^{tert}-BuOH.

In einer Variante des Verfahrens steht n für eine Zahl von 1 bis 6.

In einer Variante des Verfahrens steht n für eine Zahl von 1 bis 5.

Im Folgenden soll die Erfindung anhand von Ausführungsbeispielen näher erläutert werden.

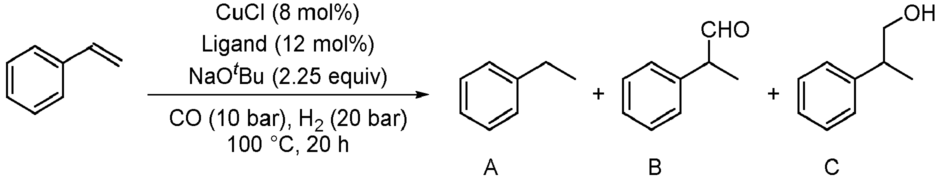

Ein getrocknetes 4-mL-Schraubdeckelgefäß, das mit einem Septum und einem Rührstab ausgestattet war, wurde mit CuCI (3,5 mg, 8 mol%), Ligand (12 mol%) und NaO^{t}Bu (95 mg, 2,25 äquiv) gefüllt. Das Fläschchen wurde mit einem PTFE/Weißgummi-Septum (Wheaton^{®}, 13 mm) und einer Phenolkappe verschlossen, mit einer kleinen Kanüle an die Atmosphäre angeschlossen, evakuiert und mit Argon aufgefüllt. Cyclohexan (1,8 mL) und Toluol (0,2 mL) wurden per Spritze unter Argon-Gegenstrom zugegeben. Zu dieser Suspension wurde das Alken (in diesem Fall Styrol) (50 µL, 0,43 mmoL) über eine Hamilton^{®} -Spritze zugegeben. Das Fläschchen wurde auf eine Metallplatte gestellt und in einen 300 mL Autoklaven (Serie 4560 von Parr instrument company^{®}) überführt. Der Autoklav wurde einmal mit Stickstoff (10 bar) und drei Mal mit CO (10 bar) gespült. Anschließend wurde der Autoklav mit CO (10 bar) und anschließend mit H₂ (20 bar) befüllt, um den gewünschten Druck von 30 bar zu erreichen. Der Autoklav wurde dann in einen Aluminiumblock auf einen Magnetrührer gestellt. Das Reaktionsgemisch wurde 20 h lang bei 100 °C gerührt (600 U/min). Anschließend wurde es auf Raumtemperatur abgekühlt und der Druck vorsichtig abgelassen. Die Reaktion wurde mit gesättigter NH₄Cl-Lösung (3,0 mL) gequencht, mit Et₂O drei Mal extrahiert, mit Salzlösung gewaschen, über Na₂SO₄ getrocknet und im Vakuum konzentriert. Der Rückstand wurde durch Gradientensäulenchromatographie (Pentan:EtOAc = 9:1 → 3:1) gereinigt, um Produkt als farbloses Öl zu erhalten (37,4 mg, 64 %).

Der Versuch wurde unter analogen Bedingungen mit den folgenden Liganden durchgeführt: 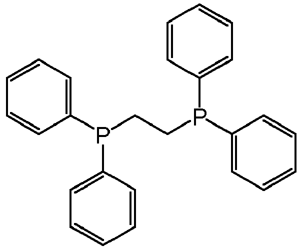
**(1)** (1,2-Bis(diphenylphosphino)ethan (DPPE)) 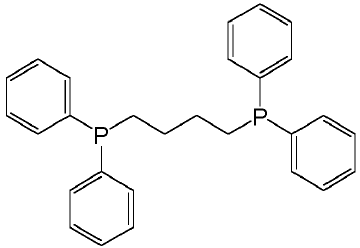
**(2)** (1,4-Bis(diphenylphosphino)butan (DPPB)) 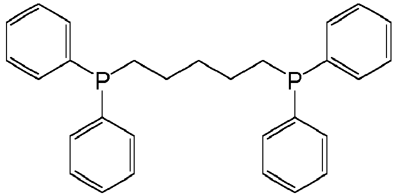
**(3)** (1,5-Bis(diphenylphosphino)pentan (DPPPE)) 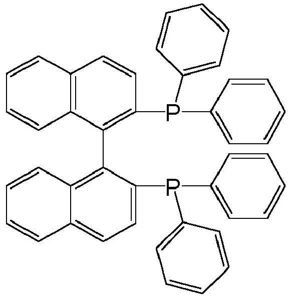
**(4)** (2,2'-Bis(diphenylphosphino)-1,1'-binaphthyl (BINAP)) 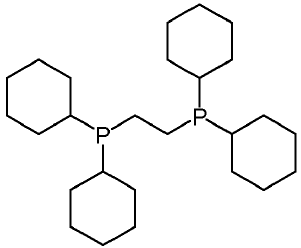
**(5)** (1,2-Bis(dicyclohexylphosphino)ethan (DCyPE)) 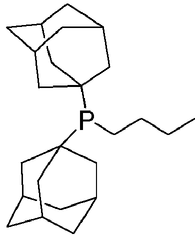
**(6)** (Butyldi-1-adamantylphosphine (BuPAd₂))

Bei den Liganden **(4), (5)** und **(6)** handelt es sich um Vergleichsliganden.

### Reaktionsbedingungen:

T = 100 °C, p(CO) = 10 bar, p(H₂) = 20 bar, t = 20 h, Lösungsmittel = Cyclohexan:Toluol (9:1)

Die Versuchsergebnisse sind in der nachfolgenden Tabelle aufgeführt:

| Ligand | Ausbeute B + C [%] |
|---|---|
| **(1)*** | 28 |
| **(2)*** | 51 |
| **(3)*** | 26 |
| **(4)** | 0 |
| **(5)** | 3 |
| **(6)** | 3 |

| | |
|---|---|
| * erfindungsgemäßes Verfahren | |

Mit den erfindungsgemäßen Verfahren konnten bessere Ausbeuten erzielt werden als in den Vergleichsversuchen.

Wie die Ausführungsbeispiele zeigen, wird die Aufgabe durch die erfindungsgemäßen Verfahren gelöst.

## Patentansprüche

1. Verfahren umfassend die Verfahrensschritte:
a) Vorlegen eines Alkens;
b) Zugabe eines Liganden gemäß Formel **(I):** 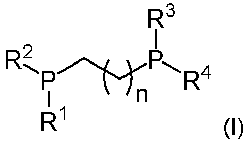 wobei
R¹, R², R³, R⁴ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -(C₅-C₂₀)-Heteroaryl,
und n für eine Zahl von 1 bis 8 steht;
c) Zugabe einer Verbindung, welche Cu umfasst;
d) Zuführen von CO und H₂;
e) Erwärmen des Reaktionsgemisches aus a) bis d), wobei das Alken zu einem Aldehyd und/oder Alkohol umgesetzt wird.

2. Verfahren nach Anspruch 1,
wobei R¹, R² für -(C₆-C₂₀)-Aryl stehen.

3. Verfahren nach einem der Ansprüche 1 oder 2,
wobei R¹, R² für -Ph stehen.

4. Verfahren nach einem der Ansprüche 1 bis 3,
wobei R¹, R³ für -(C₆-C₂₀)-Aryl stehen.

5. Verfahren nach einem der Ansprüche 1 bis 4,
wobei R¹, R³ für -Ph stehen.

6. Verfahren nach einem der Ansprüche 1 bis 5,
wobei der Ligand eine der Strukturen **(1), (2), (3)** aufweist:

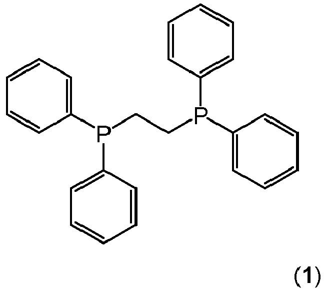

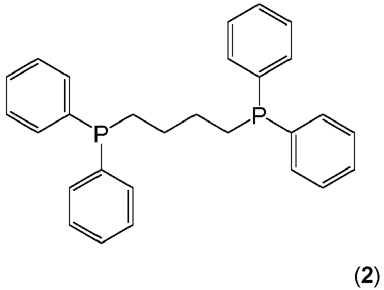

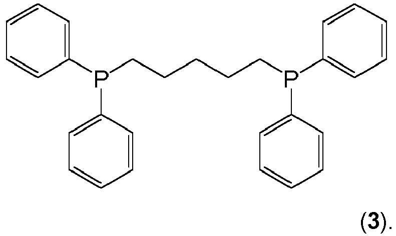

7. Verfahren nach einem der Ansprüche 1 bis 6,
wobei die Verbindung, welche Cu umfasst, ausgewählt ist aus: CuCl₂, CuBr₂, Cu(OAc)₂, Cu(OTf)₂, CuCI, CuBr, Cul, Cul(MeSMe).

8. Verfahren nach einem der Ansprüche 1 bis 7,
wobei das Zuführen von CO in Verfahrensschritt d) mit einem Druck in dem Bereich von 0,5 bis 5 MPa (5 bis 50 bar) erfolgt.

9. Verfahren nach einem der Ansprüche 1 bis 8,
wobei das Zuführen von H₂ in Verfahrensschritt d) mit einem Druck in dem Bereich von 1 bis 5 MPa (10 bis 50 bar) erfolgt.

10. Verfahren nach einem der Ansprüche 1 bis 9,
wobei das Erwärmen des Reaktionsgemisches in Verfahrensschritt e) auf eine Temperatur in dem Bereich von 70 °C bis 150 °C erfolgt.

11. Verfahren nach einem der Ansprüche 1 bis 10,
umfassend den zusätzlichen Verfahrensschritt d'):
d') Zugabe einer Base.

12. Verfahren nach Anspruch 11,
wobei die Base ausgewählt ist aus: KO^{t}Bu, NaO^{t}Bu, KOMe, NaOEt, DBU, NEt₃.

13. Verfahren nach einem der Ansprüche 1 bis 12,
umfassend den zusätzlichen Verfahrensschritt d"):
d") Zugabe eines Lösungsmittels.

14. Verfahren nach Anspruch 13,
wobei das Lösungsmittel ausgewählt ist aus: Cyclohexan, Toluol, CH₃CN, DMSO, DMF, ^{tert}-BuOH.

15. Verfahren nach einem der Ansprüche 1 bis 14,
wobei n für eine Zahl von 1 bis 5 steht.

## Geänderte Patentansprüche

### Geänderte Patentansprüche gemäss Regel 137(2) EPÜ.

1. Verfahren umfassend die Verfahrensschritte:
a) Vorlegen eines Alkens;
b) Zugabe eines Liganden gemäß Formel (I): 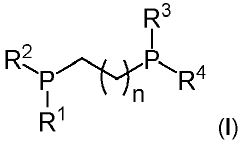 wobei
R¹, R², R³, R⁴ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -(C₅-C₂₀)-Heteroaryl,
und n für eine Zahl von 1 bis 8 steht;
c) Zugabe einer Verbindung, welche Cu umfasst, wobei die Verbindung ausgewählt ist aus: CuCl₂, CuBr₂, Cu(OAc)₂, Cu(OTf)₂, CuCl, CuBr, Cul, Cul(MeSMe);
d) Zuführen von CO und H₂;
e) Erwärmen des Reaktionsgemisches aus a) bis d), wobei das Alken zu einem Aldehyd und/oder Alkohol umgesetzt wird.

2. Verfahren nach Anspruch 1,
wobei R¹, R² für -(C₆-C₂₀)-Aryl stehen.

3. Verfahren nach einem der Ansprüche 1 oder 2,
wobei R¹, R² für -Ph stehen.

4. Verfahren nach einem der Ansprüche 1 bis 3,
wobei R¹, R³ für -(C₆-C₂₀)-Aryl stehen.

5. Verfahren nach einem der Ansprüche 1 bis 4,
wobei R¹, R³ für -Ph stehen.

6. Verfahren nach einem der Ansprüche 1 bis 5,
wobei der Ligand eine der Strukturen (1), (2), (3) aufweist:

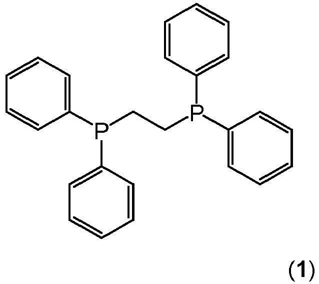

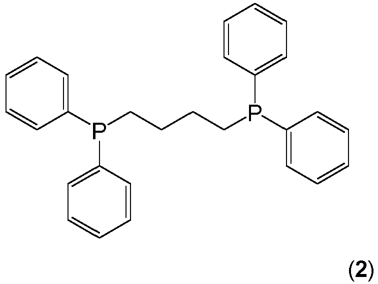

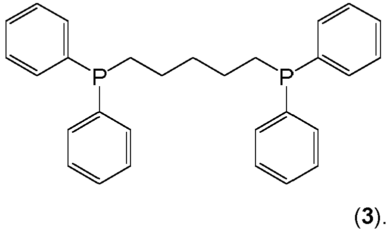

7. Verfahren nach einem der Ansprüche 1 bis 6,
wobei das Zuführen von CO in Verfahrensschritt d) mit einem Druck in dem Bereich von 0,5 bis 5 MPa (5 bis 50 bar) erfolgt.

8. Verfahren nach einem der Ansprüche 1 bis 7,
wobei das Zuführen von H₂ in Verfahrensschritt d) mit einem Druck in dem Bereich von 1 bis 5 MPa (10 bis 50 bar) erfolgt.

9. Verfahren nach einem der Ansprüche 1 bis 8,
wobei das Erwärmen des Reaktionsgemisches in Verfahrensschritt e) auf eine Temperatur in dem Bereich von 70 °C bis 150 °C erfolgt.

10. Verfahren nach einem der Ansprüche 1 bis 9,
umfassend den zusätzlichen Verfahrensschritt d'): d') Zugabe einer Base.

11. Verfahren nach Anspruch 10,
wobei die Base ausgewählt ist aus: KO^{t}Bu, NaO^{t}Bu, KOMe, NaOEt, DBU, NEt₃.

12. Verfahren nach einem der Ansprüche 1 bis 11,
umfassend den zusätzlichen Verfahrensschritt d"): d") Zugabe eines Lösungsmittels.

13. Verfahren nach Anspruch 12,
wobei das Lösungsmittel ausgewählt ist aus: Cyclohexan, Toluol, CH₃CN, DMSO, DMF, ^{tert}-BuOH.

14. Verfahren nach einem der Ansprüche 1 bis 13,
wobei n für eine Zahl von 1 bis 5 steht.
